# EUROPEAN PATENT SPECIFICATION

(11) **EP 1 871 895 B1**
(45) Date of publication and mention of the grant of the patent: **10.06.2009**
(21) Application number: 06723639.8
(22) Date of filing: 12.04.2006
(51) Int. Cl.: C12Q 1/60, C12Q 1/61

(54) **METHOD FOR SCREENING MTP-INHIBITING COMPOUNDS**
VERFAHREN ZUM SCREENING MTP HEMMENDER VERBINDUNGEN
METHODE DE CRIBLAGE DE COMPOSES D'INHIBITION D'UNE PROTEINE DE TRANSFERT DE TRIGLYCERIDE MICROSOMALE (MTP)

(30) Priority: 22.04.2005 FR 0504060
(43) Date of publication of application: 02.01.2008
(73) Proprietor: Merck Patent GmbH, 64293 Darmstadt (DE)
(72) Inventor: CHEVREUIL, Olivier, F-01400 Condeissat (FR); DUPONT, Hervé, F-69005 Lyon (FR); GUERRIER, Daniel, F-42600 Lesignieux (FR)
(74) Representative: Eiermann, Volker
(86) International application number: PCT/EP2006/002647
(87) International publication number: WO 2006/111238

(56) References cited:
- WO-A-02/42291
- US-A1- 2003 166 590
- BORRADAILE NICA M ET AL: "Hepatocyte ApoB-containing lipoprotein secretion is decreased by the grapefruit flavonoid, naringenin, via inhibition of MTP-mediated microsomal triglyceride accumulation." BIOCHEMISTRY, vol. 42, no. 5, 11 February 2003 (2003-02-11), pages 1283-1291, XP002361244 ISSN: 0006-2960
- BORRADAILE NICA M ET AL: "Inhibition of hepatocyte apoB secretion by naringenin: Enhanced rapid intracellular degradation independent of reduced microsomal cholesteryl esters" JOURNAL OF LIPID RESEARCH, vol. 43, no. 9, September 2002 (2002-09), pages 1544-1554, XP002361245 ISSN: 0022-2275
- JAMIL H ET AL: "EVIDENCE THAT MICROSOMAL TRIGLYCERIDE TRANSFER PROTEIN IS LIMITING IN THE PRODUCTION OF APOLIPOPROTEIN B-CONTAINING LIPOPROTEINS IN HEPATIC CELLS" JOURNAL OF LIPID RESEARCH, BETHESDA, MD, US, vol. 39, no. 7, July 1998 (1998-07), pages 1448-1454, XP008003194 ISSN: 0022-2275
- DIXON JOSEPH L ET AL: "Regulation of hepatic secretion of apolipoprotein B-containing lipoproteins: Information obtained from cultured liver cells" JOURNAL OF LIPID RESEARCH, vol. 34, no. 2, 1993, pages 167-179, XP002361246 ISSN: 0022-2275 cited in the application
- WETTERAU, JOHN R. ET AL: "Localization of intracellular triacylglycerol and cholesteryl ester transfer activity in rat tissues" BIOCHIMICA ET BIOPHYSICA ACTA, LIPIDS AND LIPID METABOLISM , 875 (3), 610 -17 CODEN: BBLLA6; ISSN: 0005-2760, 1986, XP002361247 cited in the application

## Description

The invention relates to a screening method for selecting specific inhibitors of microsomal triglyceride transfer protein (MTP) which are efficient and have a short duration of action.

MTP ("microsomal triglyceride transfer protein") is a transfer protein located in the reticulum of hepatocytes and enterocytes, which catalyses the assembly of biomolecules that transport triglycerides, the apoB lipoproteins.

The term apoB more particularly denotes apoprotein B48 of the intestine and apoprotein B100 of the liver, without, however, being limited thereto.

Mutations in MTP or in the B apoproteins are reflected in man by very low levels or even an absence of apoB lipoproteins. The lipoproteins containing apoB (chylomicrons, "Very Low Density Lipoproteins" = VLDL) and their metabolic residues (chylomicron remnants, "Low Density Lipoproteins" = LDL) are recognised as being a major risk factor in the development of atherosclerosis, a major cause of death in industrialised countries.

It is observed that, in individuals who are heterozygous for these mutations, levels reduced on average by a half are associated with a low cardiovascular risk (C.J. Glueck, P.S. Gartside, M.J. Mellies, P.M. Steiner, Trans. Assoc. Am. Physicians, 90, 184 (1977)).

This suggests that modulation of the secretion of triglyceride-rich lipoproteins by means of MTP antagonists and/or of secretion of apoB might be useful in the treatment of atherosclerosis and more broadly of pathologies characterised by an increase in apoB lipoproteins.

Molecules that inhibit MTP and/or the secretion of apoB might thus be useful for the treatment of diabetes-related hypertriglyceridaemia, hypercholesterolaemia and dyslipidaemia, and also for the prevention of and treating obesity.

There are already a number of examples in the literature of compounds capable of inhibiting MTP, but it is observed that since the start of the investigations on these inhibitors, none of them has become commercially available. Indeed, many development projects were stopped after the first clinical trials.

Jamil et al. (J Lipid Res 1998, 39, 1448) provided the evidence that the microsomal triglyceride transfer protein is rate limiting in the production of apolipoprotein B-containing lipoproteins in hepatic cells. Measurements of apoB concentration in the media of HepG2 cells showed that apoB secretion was reduced in proportion to the *in-situ* inhibition of MTP activity by the action of the inhibitor BMS-192951.

Borradaile et al. (J Lipid Res 2002, 43, 1544) described an enhanced rapid intracellular degradation, which is independent of reduced microsomal cholesteryl esters, following the inhibition of hepatocytes apoB secretion by the grapefruit flavonoid naringenin. Borradaile et al. (Biochem 2003, 42, 1283) have also found that the apoB-containing lipoprotein secretion is decreased by naringenin via inhibition of MTP-mediated microsomal triglyceride accumulation in HepG2 human hepatoma cells. Multicompartmental modeling of pulse-chase studies was used to analyze cellular apoB kinetics.

Dixon & Ginsberg (J Lipid Res 1993, 34, 167) addressed the regulation of hepatic secretion of apoB-containing lipoproteins. ApoB secretion is regulated post-transcriptionally and reacts rapidly to the current state of lipid metabolism in the cell. The shortage of triglyceride and/or cholesteryl ester could inhibit the secretion of apoB. ApoB secretion may be different in rat hepatocytes, HepG2 cells and human hepatocytes in-vivo.

A kit for measuring the inhibition of labeled lipid transfer from donor particles to acceptor particles in the presence of MTP is state of the art. With it, Wetterau & Zilversmit (Biochim Biophys Acta 1986. 875, 610) localized the intracellular triacylglycerol and cholesterol ester transfer activity in rat tissues. When isolated microsomes were subjected to proteolytic attack, the transfer activity was not inactivated. In particular, a screen for identifying inhibitors of MTP is disclosed in US 2003/0166590 A1. Nucleic acid sequences, particularly DNA sequences, coding for all or part of the high molecular weight subunit of microsomal triglyceride transfer protein, expression vectors containing the DNA sequences, host cells containing the expression vectors, and methods utilizing these materials are also known from US 2003/0166590 A1. The document also concerns polypeptide molecules comprising all or part of the high molecular weight subunit of microsomal triglyceride transfer protein, and methods for producing these polypeptide molecules. The document additionally concerns novel methods for preventing, stabilizing or causing regression of atherosclerosis and therapeutic agents having such activity. The document concerns further novel methods for lowering serum liquid levels and therapeutic agents having such activity.

Specifically, preliminary results of clinical studies support the inhibition of MTP as a means of reducing the level of triglycerides and of cholesterol in man. However, such a therapy requires a long treatment, extending over several years. The *in vivo* administration to man of MTP-inhibiting compounds over such long periods might give rise to toxic effects, for instance an accumulation of lipids in the intestine and the liver, leading, for example, to hepatic steatosis.

In other words, although an *in vitro* primary screening test now makes it possible to identify potential MTP-inhibfing candidates, the *in vivo* confirmation tests show that many of these candidates are responsible for toxic effects on the liver.

To overcome this problem, it is nowadays proposed, for example, to combine MTP inhibitors with fibrates. However, this type of therapy has the draw-backs associated with the administration of two active materials (problems of dosage, compatibility, etc.).

There is consequently still a need for MTP-inhibiting active substances that induce few or no toxic side effects, in particular hepatic steatosis.

An object of the invention consists in providing a screening test for selecting compounds capable of reducing hypertriglyceridaemia and cholesterolaemia with a lower risk of lipid accumulation in the liver.

Other objects will become apparent in the description of the invention discussed in further detail hereinbelow.

The present invention is based on the hypothesis that the liver resecretes the excess accumulated lipids, this excess being inherent to the inhibitory activity of the compound, since it is no longer active.

The inventors have now discovered, surprisingly, that, given that a certain potency is necessary in order to obtain a sufficient pharmacological effect, only active principles having a short duration of action and liable to give a sufficient pharmacological effect ("flash" effect) generate fewer or none of the toxic effects observed with inhibitors with a long duration of action.

In addition, since the use in man involves a chronic treatment, it is necessary for this recovery phase by the liver to take place between two administrations in order to avoid the accumulation of lipids possibly leading to the development of steatosis.

Consequently, the inventors have, for the first time, used a known qualitative test for the selection of MTP-inhibiting candidates, to determine the kinetics of action of the said candidate. In the present invention *in vivo* methods are not practised on a human body.

Thus, the present invention first proposes a screening method for selecting active materials that inhibit microsomal triglyceride transfer protein (MTP), comprising the steps of:
a) using a candidate compound in a test of kinetic monitoring of inhibition of a parameter associated with the inhibition of MTP (inhibition of secretion of apoB, inhibition of secretion of VLDL and the like);
b) monitoring of the kinetics of inhibition of the said parameter by the said candidate from the start of the test and for a duration of between 3 hours and 24 hours, preferably between 5 hours and 12 hours and more preferably between 6 hours and 10 hours; and
c) selection of the candidate if it has kinetics of inhibition of the said parameter characterised by:
   i) a percentage of inhibition for the said parameter of greater than or equal to 50% over a maximum duration of less than 4 hours and preferably less than 3 hours; and
   ii) a residual inhibitory activity for the said parameter of less than 20% and preferably less than 10%, beyond 10 hours, preferably beyond 8 hours and more preferably beyond 6 hours, after the start of the test.

In the screening method of the present invention, the test for kinetic monitoring of inhibition is referred to as "Test A" in the rest of the present description.

Thus, Test A defined above makes it possible to select compounds having both sufficient MTP-inhibiting activity, and an absence of residual inhibitory activity that might result in adverse effects, such as those mentioned above and in particular hepatic steatosis.

The term "sufficient MTP-inhibiting activity" means that the percentage of inhibition observed for the parameter associated with the inhibition of MTP (for example apoB or VLDL) is at least equal to 50%.

The term "residual inhibitory activity" should be understood as meaning inhibitory activity observed for the parameter associated with the inhibition of MTP (for example apoB or VLDL) of less than 20%, preferably less than 10%, and more preferably insignificant inhibitory activity *versus* placebo.

In the rest of the present description, the term "reversible" qualifies compounds with a short duration of action, i.e. those that have the desired "flash" effect, and "irreversible" qualifies compounds with a long duration of action.

Test A of the screening method according to the present invention allows the selection of reversible candidates.

This Test A is a kinetic monitoring of inhibition of a parameter associated with the inhibition of MTP, for example a test of inhibition of the secretion of apoprotein B (apoB), advantageously if it is an *in vitro* test, using hepatic or enteric cells of any type, preferably hepatic cells, such as HepG2 cells, or alternatively a test of kinetic analysis of inhibition of MTP on the secretion of very low density lipoproteins (VLDL), advantageously if it is an *in vivo* test. The examples that follow show, for illustrative purposes, particular modes of implementation of Test A.

Test A may be performed *in vitro* or *in vivo.* According to one preferred variant of the invention, Test A performed *in vitro* (noted as A*ᵥᵢₜᵣₒ* in the rest of the description) measures the kinetics of secretion of apoB, and Test A performed *in vivo* (noted as A*ᵥᵢᵥₒ* in the rest of the description) measures the kinetics of secretion of VLDLs.

In Test A*ᵥᵢₜᵣₒ*, the candidate compounds are considered as having a short duration of action if they show satisfactory reversibility, i.e. if the secretion of apoB, 24 hours after removal of the test compound, has returned to a level whose value is greater than or equal to 50% and advantageously greater than or equal to 70%, relative to a control that has not, by definition, been treated with an inhibitory compound.

According to one preferred variant of the present invention, Test A is performed *in vitro,* and the selected candidates are then used in one or more Tests A *in vivo,* in order to confirm the activity of the candidate selected after the Test A *in vitro.*

Test A is performed *in vitro* on cells in culture that are preferably hepatic cells, advantageously HepG2 cells. Test A is performed *in vivo* on a suitable animal model, for example rats.

In addition, Test A defined above may be combined with one or more other preselection or post-selection tests, allowing faster and/or more specific screening for the selection of MTP-inhibiting candidates with a short duration of action.

Thus, in addition to the test of reversibiliby of action on MTP (the kinetic Test A defined above), the screening method according to the invention may be followed or preceded by one or more qualitative tests of inhibition of the activity of MTP, for instance the test described by Wetterau and Zilversmit (Biochem. Biophys. Acta, (1986), 875-610).

This test (referred to as Test B hereinbelow) makes it possible to perform a qualitative selection of candidates with regard to their capacity to inhibit or otherwise MTP *in vitro.* By virtue of its nature, this test is advantageously performed before Test A defined above.

In addition, it may prove to be advantageous to use the candidate products in a test (referred to as Test C in the rest of the description) of inhibition of secretion of apoB, for example in the HepG2 human cell line, allowing analysis of the inhibitory capacity of the candidates on the secretion of apoB.

Test C is of interest, like Test B, in particular if it is used before Test A, to allow a preliminary screening of "active-inactive" type of the test compounds.

Test C is also known and commonly used in the field. Reference may be made, for example, to Dixon J. and Ginsberg H. (J. Lipid. Res., (1993), 34, 167-179) for the implementation of this test.

The screening method according to the present invention for selecting compounds with satisfactory reversibility may, according to another embodiment, also comprise one or more tests for determining whether the plasmatic metabolites of the test compounds are active or inactive.

Specifically, the problem that the present invention proposes to solve consists in selecting MTP inhibitors that have satisfactory inhibitory activity but of short duration ("flash" effect, reversible inhibitors). Consequently, it is advantageous to ensure that the plasmatic metabolites of the compounds selected or to be selected do not themselves have inhibitory activity on the same target, i.e. an IC₅₀ on MTP activity of greater than 1 µM and preferably greater than 10 µM.

Thus, Test A may also be preceded or followed, preferably followed, by one or more tests of inhibition of MTP and/or of inhibition of the secretion of VLDLs and/or of inhibition of the secretion of apoB, which are similar or even identical to the Tests B and/or C, respectively, defined above, but performed this time on the plasmatic metabolites of the candidate compounds. These tests on the metabolites will be referred to hereinbelow as Test D (metabolites/MTP inhibition test) and Test E (metabolites/apoB inhibition test).

The term "plasmatic metabolites" means the products of degradation of the candidate compounds by the live body. These metabolites are obtained according to any standard method known to those skilled in the art, for example by structural determination of the products of degradation of the candidate compounds after incubation in live cells, preferably in microsomes, followed by resyn-thesis of the potential metabolites.

The screening method according to the present invention may be followed by one or more tests of confirmation (post-selection) of *in vivo* activity. Advantageously, the screening method according to the invention will also include at least one *in vivo* confirmation test chosen from a qualitative test of inhibition of VLDL secretion, similar to the *in vitro* tests B and C defined above, and a test of the evaluation of the kinetics of inhibition of VLDL secretion, similar to the *in vitro* test A defined above. These two confirmation tests will be referred to as test F and test A*ᵥᵢᵥₒ*, respectively.

Finally, the candidates that have responded positively to the tests selected from those defined above may be subjected to a final test (Test H), *in vivo,* for determination of the desired pharmacological effect (reduction of triglycerides in the blood). This Test H may be of any known type, for example of the type presented in the examples that follow.

All of the tests described above, which complement/confirm test A in the screening method according to the invention are collated in the table below in chronological order, which constitutes a preferred variant of the screening method according to the invention, with mention of the test target, i.e. animal model (in particular rats) for the *in vivo* tests, and HepG cells, MTP proteins or any other type of liver cell for the *in vitro* tests:

| ***Test*** | ***in vitro*/*in vivo*** | ***Substrate*** | ***Target*** | ***Description*** |
|---|---|---|---|---|
| *Preselection* | | | | |
| B | *in vitro* | Candidate | MTP proteins | MTP inhibition/qualitative |
| C | *in vitro* | Candidate | HepG2 cells | apoB inhibition/qualitative |

| *Selection* | | | | |
|---|---|---|---|---|
| A*ᵥᵢₜᵣₒ* | *in vitro* | Candidate | HepG2 cells | apoB inhibition/kinetics |

| *Tests on the metabolites* | | | | |
|---|---|---|---|---|
| D | *in vitro* | Metabolites | MTP proteins | MTP inhibition/qualitative |
| E | *in vitro* | Metabolites | HepG2 cells | apoB inhibition/qualitative |

| *Post-selection -- Confirmation* | | | | |
|---|---|---|---|---|
| F | *in vivo* | Candidate | - animal - | VLDL inhibition/qualitative |
| *Aᵥᵢᵥₒ* | *in vivo* | Candidate | - animal - | VLDL inhibition/kinetics |
| H | *in vivo* | Candidate | - animal - | Desired pharmacological effect (reduction of triglycerides in the blood) |

It is clearly understood that the kinetic Test A can be used as sole test, *in vivo* or *in vitro,* for the determination of MTP-inhibiting compounds with a short duration of action. It will be noted, however, that if Test A*ᵥᵢₜᵣₒ* is used alone in the said method, it will advantageously be followed by a Test A*ᵥᵢᵥₒ* of kinetic analysis of the inhibitory power of the candidate compound.

According to one preferred embodiment, the invention relates to a method for screening MTP-inhibiting compounds, comprising the selection of candidates by means of a Test A*ᵥᵢₜᵣₒ* as defined above, followed by the selection of the candidates that have responded positively to the Test A*ᵥᵢₜᵣₒ* by means of a Test A*ᵥᵢᵥₒ*.

According to another embodiment, the invention relates to a method for screening MTP-inhibiting compounds, comprising a preselection of candidates chosen from those that have responded positively to Test B and/or to Test C defined above, selection from the candidates derived from the preselection by means of a Test A*ᵥᵢₜᵣₒ* as defined above, and then selection of the candidates that have responded positively to Test A*ᵥᵢₜᵣₒ* by means of a Test A*ᵥᵢᵥₒ*.

According to yet another embodiment, the present invention relates to a screening method comprising the steps of:
a) using candidate compounds in a Test B and/or a Test C, followed by preselection of the candidates responding positively to the said Test B and/or to the said Test C;
b) using the candidate compounds derived from step a) in a Test A, and advantageously a Test A*ᵥᵢₜᵣₒ,* and selection of the candidates responding positively to the said Test A;
c) optional additional selection of the candidate compounds selected from step b), via analysis of the metabolites of the said candidates by means of a Test D and/or a Test E;
d) using the candidate compounds selected during steps a), b) and c) in at least one *in vivo* confirmation test F and/or A*ᵥᵢᵥₒ,* followed by selection of the candidates responding positively to Test F and/or to Test A*ᵥᵢᵥₒ*; and
e) confirmation of the candidates selected during the preceding step, by selection using an *in vivo* Test H of control of the reduction of triglycerides in the blood.

The implementation of the various tests described above is preferably performed in the order B and/or C - A*ᵥᵢₜᵣₒ* - D and/or E - F and/or A*ᵥᵢᵥₒ* - H, without this order constituting any limitation of the present invention.

The present invention also relates to the use of the screening method according to the invention comprising at least one Test A*ᵥᵢₜᵣₒ*, a Test A*ᵥᵢᵥₒ* or a combination of the two, as defined above, to determine the kinetics of a parameter associated with the inhibition of MTP by a candidate compound. The conditions for performing these tests are those described in the screening method.

Preferably, this test is used to determine whether the candidate compound has a short duration of action ("flash" effect) within the meaning of the present invention, or, on the contrary, a long duration of action.

The compounds selected via the screening method according to the invention, have an entirely advantageous possible application in the treatment of hypertriglyceridaemia, hypercholesterolaemia and dyslipidaemia associated with metabolic syndrome and diabetes, and pancreatitis, but also for the prevention of and treating obesity.

Thus, the MTP inhibitors with a short duration of action selected via the screening method according to the present invention induce a significant reduction in hypertriglyceridaemia in obese Zucker rats (fatty fa/fa), an animal model of hypertriglyceridaemia, without aggravating the lipid content of the liver (steatosis), as is observed, on the contrary, with an MTP inhibitor with a long duration of action.

For purely illustrative purposes, compounds 1 and 2 described in patent application FR 2 816 940 (and WO 02/42291) are MTP-inhibiting compounds with a short duration of action.

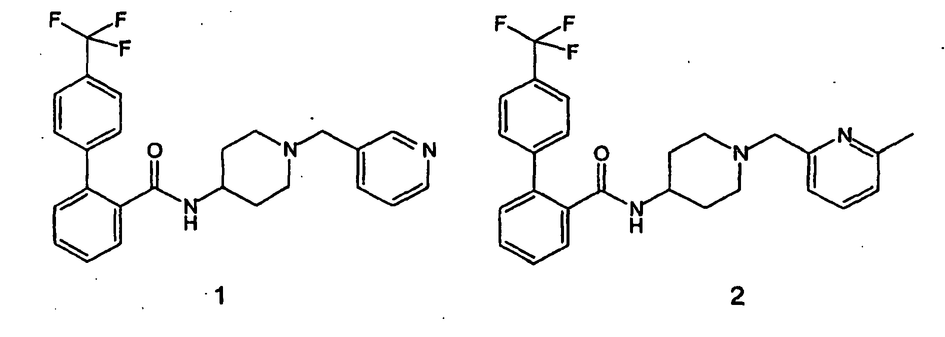

The invention will now be described in greater detail with the aid of the modes of implementation taken as non-limiting examples and with reference to the drawing, in which:
- Figure 1 is a graph with, on the x-axis, the time (minutes) after treating rats with the abovementioned compound 1, and, on the y-axis, the percentage of inhibition, illustrating the duration of action of compound 1 on the secretion of VLDL-triglycerides;
- Figure 2 is a graph with, on the x-axis, the time (minutes) after treating rats with the abovementioned compound 2, and, on the y-axis, the percentage of inhibition, illustrating the duration of action of compound 2 on the secretion of VLDL-triglycerides.

### EXAMPLES

### TEST B

### Qualitative analysis of the inhibition of MTP activity in vitro

The inhibition of the activity of microsomal triglyceride transfer protein (MTP) was tested by using the following operating protocol.

The inhibition of MTP activity with a compound may be quantified by observing the inhibition of the transfer of a labelled triglyceride, from a donor particle to an acceptor particle, in the presence of MTP. The procedure for the preparation of MTP is based on the method by Wetterau and Zilversmit (Biochem. Biophys. Acta (1986) 875, 610). A few grams of golden hamster liver are taken and then rinsed several times in a 250 mM sucrose solution at 0°C. All the following steps proceed at +4°C. A homogenate at a concentration of 50% in 250 mM sucrose is prepared using a Teflon mill and then centrifuged for 10 minutes at 10 000xg at +4°C. The supernatant is then centrifuged at 105 000xg for 75 minutes at +4°C. The supernatant is discarded and the microsomal pellet is taken up in 3 ml (per g of starting liver) of Tris/HCl 150 mM pH 8.0. 1 ml aliquot fractions are stored at -80°C until the time of use.

After thawing a fraction of microsomes (1 ml), 12 ml of refrigerated Tris/HCl 50 mM, KCI 50 mM, MgCl₂ 5 mM pH 7.4 buffer and 1.2 ml of deoxycholate (0.54% in water) are added. After incubation for 30 minutes at +4°C with gentle agitation, the suspension is centrifuged at 105 000xg for 75 minutes. The supernatant comprising the soluble MTP is dialysed against Tris/HCl 150 mM, NaCl 40 mM, EDTA 1 mM, 0.02% sodium azide pH 7.4 buffer (5 times one litre over 2-3 days). The MTP is stored at +4°C, is stable for at least 30 days and is used in unmodified form in the test.

The donor particles (liposomes) are prepared from 208 µl of L-phosphatidylcholine at a concentration of 10 mg/ml in chloroform, and 480 µl of [3H]-triolein at a concentration of 0.5 mCi/ml in toluene. After stirring, the solution is evaporated under nitrogen, taken up in 6 ml of Tris/HCl 50 mM, KCI 50 mM, MgCl₂ 5 mM pH 7.4 buffer and incubated in an ultrasound bath for 30 minutes at room temperature. The liposomes are stored at +4°C and sonicated again for 10 minutes before each use.

The acceptor particles are biotinylated low density lipoproteins (LDL-biot). These particles are supplied by the company Amersham.

The reaction mixture is prepared in untreated ½ well white plates (Corning Costar) by addition, in the following order, of: 5 µl of HEPES 50 mM, NaCl 150 mM, BSA 0.1% (w/v), 0.05% sodium azide (w/v), pH 7.4 buffer, 5 µl of liposomes; 5 µl of LDL-biot; 5 µl of test products in DMSO; 5 µl of MTP. After incubation for 18-24 hours at 37°C, the reaction is stopped by adding 100 µl of Amersham SPA (Scintillation Proximity Assay) beads coupled to streptavidin, and the radioactivity is counted using a Top Count (Packard) machine at least one hour later. The inhibition of the transfer of the triglycerides with a compound is reflected by a reduction in the transferred radioactivity. The percentage of inhibition for a given compound is determined relative to controls that do not comprise compounds in the reaction mixture.

The results are expressed in terms of the IC₅₀, i.e. the concentration that allows a 50% inhibition of MTP. These results are summarised in Table I below for compounds 1 and 2 described above.

**--TABLE I --**

| ***Compound*** | ***IC₅₀ (nM)*** |
|---|---|
| **1** | 320 |
| **2** | 65 |

### TEST C

### Qualitative analysis of the secretion of apoB in the HepG2 human cell line:

The activity of a compound may be evaluated by measuring the inhibition of apoB secretion in HepG2 cells.

The HepG2 cells (ECACC - No. 85011430) are used as model in the study of the *in vitro* hepatic secretion of lipoproteins, as described by Dixon J. and Ginsberg H. (J. Lipid. Res., (1993), 34,167-179).

The HepG2 cells are cultured in Dulbecco's modified Eagle's medium comprising 10% foetal calf serum (DMEM and FBS - Gibco) in 96-well plates under an atmosphere of 5% carbon dioxide for 24 hours (about 70% confluence).

The test compounds are dissolved at a concentration of 2 or 10 mM in dimethyl sulfoxide (DMSO). Serial dilutions (1:3.16) are made in DMSO and are added (1:200 - Robot Multimek Beckman) to the growth medium (200 µl) and then finally incubated for 24 hours in the various wells containing the HepG2 cells.

The 24-hour culture supernatant diluted to 1:5 (phosphate-buffered saline: PBS comprising 1% bovine serum albumin) is tested according to a sandwich-ELISA method specific for human apoB.

The results, presented in Table II below for compounds 1 and 2, are expressed in terms of IC₅₀, i.e. the concentration that produces a 50% inhibition of apoB secretion in the HepG2 cells.

**-- TABLE II --**

| ***Compound*** | ***IC₅₀** (nM)* |
|---|---|
| **1** | 18.0 |
| **2** | 3.1 |

### TEST Aᵥᵢₜᵣₒ

### Analyse of the kinetics (reversibility) of inhibition of secretion of apoB by HepG2 cells:

HepG2 cells are placed in contact with the products as described in the preceding paragraph and the secretion of ApoB is measured after 6 and 24 hours. The comparison is made at equipotent dose for the inhibition of secretion of apoB after 24 hours.

Next, the products are removed from the culture medium (during changes of the medium at 0, 6, 24 and 48 hours) and the secretion of ApoB is again measured to determine the kinetics for return to normal secretion. The reversibility is judged satisfactory if the secretion, 24 hours after removal of the products, has a value greater than or equal to 70% of that of the controls (blank cells without compound).

These results are collated in Table III below for compounds 1 and 2.

**-- TABLE III --**

| ***Compound*** | ***Secretion of apoB 24 hours after removal of the products (% of the controls)*** |
|---|---|
| **1** | >85% |
| **2** | 85% |

### TEST D

### Metabolisation/inactivation of the product

The products are evaluated for their capacity to be inactivated rapidly in *vitro.* To do this, the compounds that showed satisfactory activity *in vitro* are tested for their rate of metabolisation in rodent liver microsomes. The incubation medium is prepared by mixing together the following constituents: NAD (1 mM), glucose 6 phosphate (5 mM), NADP (1 mM), glucose 6 phosphate dehydrogenase (1 IU/ml), bovine serum albumin (1 mg/ml), TRIS (10 mM pH = 7.6), MgCl₂ (5 mM).

A concentration of 0.5 mg/ml of microsomes (Bioprédic) is placed in contact with 10 µM of test product. The mixture is incubated for 1 hour at 37°C_{.} A fraction of the preparation is taken at the initial time, to which is added an equivalent volume of ethanol (quenching of the reaction by precipitation of the proteins). The same operation is repeated after 1 hour of incubation.

The samples obtained are centrifuged at 4000 rpm for 10 minutes, and the supernatant is analysed by LC/MS/MS. The percentage of disappearance of the parent product between TO and T60 minutes is determined by calculating the areas of the chromatographic peaks.

Potential metabolites M1 and M2 (of the compounds 1 or 2, respectively) are identified, resynthesised and evaluated for their MTP-inhibiting activity. Compounds 1 and 2 show (Table IV) metabolisation/inactivation of greater than 50% per hour into sparingly active or inactive metabolites (IC₅₀ > 1 µM).

**-- TABLE IV --**

| **Compound** | ***Metabolisation (%*/*hour)*** | ***Activity of the metabolites*** |
|---|---|---|
| **1** | 88 | M1, M2 inactive |
| **2** | 95 | M1, M2 inactive |

### TEST F

### Inhibition of secretion of VLDL in rats:

The inhibition of hepatic MTP by compounds 1 and 2 was evaluated from their impact on the secretion of very low density lipoproteins (VLDLs). Male Wistar rats (220-280 g) receive a standard diet and water *ad libitum.* On the day of the experiment, the animals (n = 7) are fasted and, one hour later, are treated with the compounds (100 mg/kg orally; excipient 0.5% methylcellulose). The secretion of VLDL is measured one hour after treatment, and for 5 hours, via the triton technique (WR 1339). When it is injected into the bloodstream, triton (400 mg/kg) prevents the catabolism of the VLDLs by inhibiting their lipolysis via lipoprotein lipase and by inhibiting their uptake by tissues. Measurement of the accumulation of triglycerides (TG) and of cholesterol, which is a component of VLDLs, thus makes it possible to reveal the secretion of VLDL by the liver.

The effect of the product on the secretion of VLDL is thus evaluated between 1 hour and 6 hours after treatment at a dose of 100 mg/kg, i.e. between 0 and 5 hours after the intravenous injection of triton. Blood samples are taken at regular intervals under gaseous anaesthesia, to assay of the triglycerides and the cholesterol. The secretion of VLDL-triglycerides and of VLDL-cholesterol is expressed in mmol/I/h (mean values). The levels of inhibition relative to controls not treated with the compounds are determined, and the results are collated in Table V.

**-- TABLE V --**

| ***Compound*** | ***Inhibition of secretion of VLDL-TG*** | ***Inhibition of secretion of VLDL- cholesterol*** |
|---|---|---|
| **1** | -56% | -45% |
| **2** | -49% | -46% |

### TEST Aᵥᵢᵥₒ

### Determination of the duration of action of MTP inhibitors on the secretion of VLDL

This test makes it possible to evaluate the kinetics of inhibition of MTP-inhibiting compounds on the secretion of VLDL in fatty fa/fa Zucker rats (10-12 weeks old), which is an animal model characterised by hypersecretion of VLDL. The intensity and duration of inhibition are measured via the triton technique described above over a period ranging up to 8 hours after administration of the products. Since the duration of action of a product depends both of the administered dose and on its intrinsic characteristics, it is determined at a dose of makes it possible to reach a maximum inhibition (Iₘₐₓ) of at least 50% over a significant time interval. The comparison of the profile of duration of action of two compounds that may have different potentials is thus performed at respective doses that allow similar iₘₐₓ values to be obtained. Compounds 1 and 2 show a duration of action of less than 8 hours with an absence of significant residual inhibition between 6 and 8 hours after treatment.

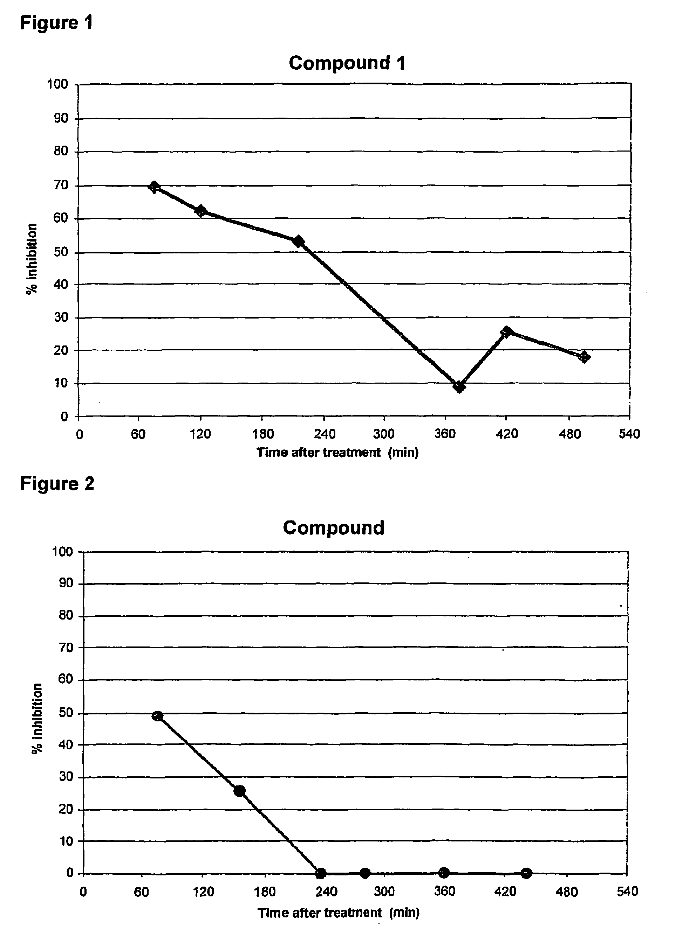

The curves of inhibition as a function of time for compounds 1 and 2 are shown in Figures 1 and 2, respectively:

### TEST H

### In vivo evaluation of the antilipaemic effect versus the steatogenic effect of MTP inhibitors

To characterise the steatogenic capacity of MTP inhibitors, a ratio between the desired pharmacological effect (reduction of triglyceridaemia or reduction of the VLDL fraction) and the potentially deleterious mechanistic effect (accumulation of triglycerides in the liver) is determined after a repeated treatment in the animal.

10-week-old fatty (fa/fa) Zucker rats are fed with a standard diet *ad libitum* and treated orally for 7 days (n = 7-8) with compound 2 (excipient 0.5% methylcellulose). The comparison between compounds with different durations of action is made at equipotent doses for the desired pharmacological effect, i.e. reduction of the circulating triglycerides, and relates to the degree of accumulation of triglycerides in the liver relative to the control animals. The results are presented in Table VI:

**-- TABLE VI --**

| ***Compounds*** | ***Plasmatic triglycerides (mM)*** | ***Variation*/*placebo (%*; *statistical)*** | ***Hepatic triglycerides (mg*/*g tissue)*** | ***Variation*/*placebo (%; statistical)*** |
|---|---|---|---|---|
| Placebo | 4.29 ± 0.13 | - | 54 ± 10 | - |
| 2 (80 mg/kg/day) | 1.40 ± 0.06 | -67%, p<0.01 | 68 ± 15 | +28%, NS |

| | | | | |
|---|---|---|---|---|
| NS: not significant Statistical: Mann-Whitney U test | | | | |

## Claims

1. Screening method for selecting active materials that inhibit microsomal triglyceride transfer protein (MTP), comprising the steps of:
a) using a candidate compound in a test of kinetic monitoring of a parameter associated with the inhibition of MTP (Test A), wherein an *in vivo* test is not practised on a human body;
b) monitoring of the kinetics of inhibition of the said parameter by the said candidate from the start of the test and for a duration of between 3 hours and 24 hours, preferably between 5 hours and 12 hours and more preferably between 6 hours and 10 hours; and
c) selection of the candidate if it has kinetics of inhibition of the said parameter characterise by:
i) a percentage of inhibition for the said parameter of greater than or equal to 50% over a maximum duration of less.than 4 hours and preferably less than 3 hours; and
ii) a residual inhibitory activity for the said parameter of less than 20% and preferably less than 10%, beyond 10 hours, preferably beyond 8 hours and more preferably beyond 6 hours, after the start of the test.

2. Method according to Claim 1, **characterised in that** it is performed *in vitro* or *in vivo,* preferably *in vitro* and more preferably *in vitro* and then *in vivo.*

3. Method according to Claim 1, **characterised in that** the parameter associated with inhibition of MTP is the inhibition of secretion of apoprotein B (apoB), using hepatic or enteric cells of any type, such as HepG2 cells, if it is an *in* vitro test, and inhibition of MTP on the secretion of very low density lipoproteins (VLDL), if it is an *in vivo* test.

4. Method according to any one of Claims 1 to 3, **characterised in that** the kinetic monitoring test (Test A) is preceded or followed by an in vitro test of inhibition of the activity of MTP (Test B).

5. Method according to any one of the preceding claims, **characterised in that** the kinetic monitoring test is preceded or followed by an in vitro test of inhibition of the secretion of apoB (Test C).

6. Method according to any one of the preceding claims, **characterised in that** it comprises one or more in vitro tests of inhibition of MTP (Test D) and/or of inhibition of secretion of apoB (Test E), performed on the metabolites of the candidate compounds.

7. Method according to any one of the preceding claims, **characterised in that** it is performed *in vitro* and is followed by one or more tests for confirmation of *in vivo* activity, wherein the *in vivo* tests are not practised on a human body.

8. Method according to any one of the preceding claims, **characterised in that** it comprises at least one *in vivo* confirmation test chosen from a qualitative test of inhibition of secretion of VLDL (Test F), and a test for evaluation of the kinetics of inhibition of the secretion of VLDL (Test A*ᵥᵢᵥₒ*), wherein Test F and Test A*ᵥᵢᵥₒ* are not practised on a human body.

9. Method according to any one of the preceding claims, comprising the steps of:
a) using candidate compounds in the Test B and/or the Test C, followed by preselection of the candidates responding positively to the said Test B and/or to the said Test C;
b) using the candidate compounds derived from step a) in the Test A, advantageously an *in vitro* test for measuring the kinetics of secretion of apoB (Test A*ᵥᵢₜᵣₒ*), and selection of the candidates responding positively to the said Test A;
c) optional additional selection of the candidate compounds selected from step b), via analysis of the metabolites of the said candidates by means of the Test D and/or the Test E;
d) using the candidate compounds selected during steps a), b) and c) in at least one *in vivo* confirmation test F and/or A*ᵥᵢᵥₒ*, followed by selection of the candidates responding positively to Test F and/or to Test A*ᵥᵢᵥₒ*; and
e) confirmation of the candidates selected during the preceding step, by selection using an *in vivo* test of control of the reduction of triglycerides in the blood (Test H), wherein Test H is not practised on a human body.

10. Use of the screening method according to any one of the preceding claims, comprising at least one Test A*ᵥᵢₜᵣₒ*, zest A*ᵥᵢᵥₒ* or a combination thereof, to determine the kinetics of a parameter associated with the inhibition of MTP by a candidate compound.

## Patentansprüche

1. Screeningverfahren zur Auswahl von aktiven Materialien, die das Mikrosomale Triglycerid-Transferprotein (MTP) inhibieren, umfassend die folgenden Schritte:
a) Verwendung einer Kandidatenverbindung in einem Test zur kinetischen Überwachung eines mit der Inhibierung von MTP zusammenhängenden Parameters (Test A), wobei ein *in*-*vivo*-Test nicht am menschlichen Körper praktiziert wird;
b) Überwachung der Inhibierungskinetik dieses Parameters durch diesen Kandidaten vom Beginn des Tests und für eine Dauer zwischen 3 Stunden und 24 Stunden, vorzugsweise zwischen 5 Stunden und 12 Stunden und noch mehr bevorzugt zwischen 6 Stunden und 10 Stunden; und
c) Auswahl des Kandidaten, wenn er eine Inhibierungskinetik dieses Parameters aufweist, die **gekennzeichnet ist durch**:
i) einen Prozentsatz an Inhibierung für diesen Parameter von mehr als oder gleich 50 % über eine maximale Dauer von weniger als 4 Stunden und vorzugsweise weniger als 3 Stunden; und
ii) eine verbleibende Inhibitoraktivität für diesen Parameter von weniger als 20 % und vorzugsweise weniger als 10 % nach mehr als 10 Stunden, vorzugsweise nach mehr als 8 Stunden und noch mehr bevorzugt nach mehr als 6 Stunden nach dem Testbeginn.

2. Verfahren nach Anspruch 1, **dadurch gekennzeichnet, dass** es *in vitro* oder *in vivo,* vorzugsweise *in vitro* und mehr bevorzugt *in vitro* und dann *in vivo* durchgeführt wird.

3. Verfahren nach Anspruch 1, **dadurch gekennzeichnet, dass** der mit der Inhibierung von MTP zusammenhängende Parameter die Inhibierung der Sekretion von Apoprotein B (apoB) unter Verwendung von hepatischen oder enteralen Zellen eines beliebigen Typs wie z. B. HepG2-Zellen ist, wenn es sich um einen *in*-*vitro*-Test handelt, und die Inhibierung von MTP bei Sekretion von Lipoproteinen sehr geringer Dichte (VLDL, very low density lipoproteins), wenn es sich um einen *in*-*vivo*-Test handelt.

4. Verfahren nach einem der Ansprüche 1 bis 3, **dadurch gekennzeichnet, dass** dem kinetischen Überwachungstest (Test A) ein *in*-*vitro*-Test der Aktivitätsinhibierung von MTP (Test B) vorausgeht oder ihm folgt.

5. Verfahren nach einem der vorhergehenden Ansprüche, **dadurch gekennzeichnet, dass** dem kinetischen Überwachungstest ein *in*-*vitro*-Test der Sekretionsinhibierung von apoB (Test C) vorausgeht oder ihm folgt.

6. Verfahren nach einem der vorhergehenden Ansprüche, **dadurch gekennzeichnet, dass** es einen oder mehrere *in*-*vitro*-Tests zur Inhibierung von MTP (Test D) und/oder zur Sekretionsinhibierung von apoB (Test E) umfasst, die an den Metaboliten der Kandidatenverbindungen durchgeführt werden.

7. Verfahren nach einem der vorhergehenden Ansprüche, **dadurch gekennzeichnet, dass** es *in vitro* durchgeführt wird und dass ihm ein oder mehrere Tests zur Bestätigung der *in*-*vivo*-Aktivität folgen, wobei die *in*-*vivo*-Tests nicht an einem menschlichen Körper praktiziert werden.

8. Verfahren nach einem der vorhergehenden Ansprüche, **dadurch gekennzeichnet, dass** es mindestens einen *in*-*vivo*-Bestätigungstest umfasst, ausgewählt aus einem qualitativen Test zur Sekretionsinhibierung von VLDL (Test F), und einem Test zur Evaluierung der Inhibierungskinetik der VLDL-Sekretion (Test Aᵥᵢᵥₒ), wobei Test F und Test Aᵥᵢᵥₒ nicht an einem menschlichen Körper praktiziert werden.

9. Verfahren nach einem der vorhergehenden Ansprüche, umfassend die folgenden Schritte:
a) Verwendung von Kandidatenverbindungen in einem Test B und/oder in einem Test C, gefolgt von einer Vorauswahl von Kandidaten, die positiv auf diesen Test B und/oder diesen Test C reagieren;
b) Verwendung der aus Schritt a) stammenden Kandidatenverbindungen in einem Test A, vorzugsweise in einem *in-vitro-Test* zur Messung der Sekretionskinetik von apoB (Test A*ᵥᵢₜₒᵣ*), sowie Auswahl der Kandidaten, die positiv auf diesen Test A reagieren;
c) Optionale zusätzliche Auswahl von aus Schritt b) ausgewählten Kandidatenverbindungen mittels Analyse der Metaboliten dieser Kandidaten mittels des Tests D und/oder des Tests E;
d) Verwendung der in den Schritten a), b) und c) ausgewählten Kandidatenverbindungen in mindestens einem *in*-*vivo*-Bestätigungstest F und/oder A*ᵥᵢᵥₒ*, gefolgt von der Auswahl der Kandidaten, die positiv auf Test F und/oder auf Test A*ᵥᵢᵥₒ* reagieren; und
e) Bestätigung der während des vorhergegangenen Schritts ausgewählten Kandidaten durch Auswahl unter Verwendung eines *in*-*vivo*-Tests H zur Kontrolle der Triglyceridverminderung im Blut (Test H), wobei Test H nicht an einem menschlichen Körper praktiziert wird.

10. Verwendung des Screeningverfahrens nach einem der vorhergehenden Ansprüche, umfassend mindestens einen Test A*ᵥᵢₜᵣₒ*, einen Test A*ᵥᵢᵥₒ* oder eine Kombination davon, um die Kinetik eines mit der Inhibierung von MTP zusammenhängenden Parameters mittels einer Kandidatenverbindung zu bestimmen.

## Revendications

1. Méthode de criblage pour la sélection de matières actives inhibitrices de la protéine microsomale de transfert des triglycérides (MTP), comprenant les étapes de :
a) engagement d'un composé candidat dans un test de suivi cinétique d'un paramètre lié à l'inhibition de la MTP (Test A), où un test *in vivo* n'est pas pratiqué sur le corps humain ;
b) suivi de la cinétique d'inhibition dudit paramètre par ledit candidat depuis le départ du test et pendant une durée comprise entre 3 heures et 24 heures, de préférence entre 5 heures et 12 heures, de préférence encore entre 6 heures et 10 heures ; et
c) sélection du candidat dans la cas où il présente une cinétique d'inhibition dudit paramètre **caractérisée par** :
i) un pourcentage d'inhibition pour ledit paramètre supérieur ou égal à 50% pendant une durée maximale inférieure à 4 heures, de préférence inférieure à 3 heures ; et
ii) une activité inhibitrice rémanente pour ledit paramètre inférieure à 20%, de préférence inférieure à 10%, au-delà de 10 heures, de préférence au-delà de 8 heures, de préférence encore au-delà de 6 heures, après le début du test.

2. Méthode selon la revendication 1, **caractérisée en ce qu'**elle est réalisée *in vitro,* ou *in vivo,* de préférence *in vitro,* de préférence encore *in vitro,* puis *in vivo.*

3. Méthode selon la revendication 1, **caractérisée en ce que** le paramètre lié à l'inhibition de la MTP est l'inhibition de la sécrétion d'apoprotéine B (apoB), à partir de tout type de cellules hépatiques ou entériques, telles que des cellules HepG2, lorsqu'il s'agit d'un test *in vitro,* et l'inhibition de la MTP sur la sécrétion de lipoprotéines de très basses densité ("Very Low Density Liporoteins" = VLDL), lorsqu'il s'agit d'un test in *vivo*.

4. Méthode selon l'une quelconque des revendications 1 à 3, **caractérisée en ce que** le test de suivi cinétique (Test A) est précédé ou suivi d'un test *in vitro* d'inhibition de l'activité de la MTP (Test B).

5. Méthode selon l'une quelconque des revendications précédentes, **caractérisée en ce que** le test de suivi cinétique est précédé ou suivi d'un test d'inhibition de la sécrétion d'apoB *in vitro* (Test C).

6. Méthode selon l'une quelconque des revendications précédentes, **caractérisée en ce qu'**elle comprend un ou plusieurs tests *in vitro* d'inhibition de la MTP (Test D) et/ou d'inhibition de la sécrétion d'apoB (Test E), réalisés sur les métabolites des composés candidats.

7. Méthode selon l'une quelconque des revendications précédentes, **caractérisée en ce qu'**elle est réalisée *in vitro* et est suivie d'un ou plusieurs tests de confirmation d'activité *in vivo,* où les tests *in vivo* ne sont pas pratiqué sur le corps humain.

8. Méthode selon l'une quelconque des revendications précédentes, **caractérisée en ce qu'**elle comprend au moins un test de confirmation *in vivo* choisi parmi un test qualitatif d'inhibition de la sécrétion des VLDL (Test F), et un test d'évaluation de la cinétique d'inhibition de la sécrétion des VLDL (Test A*ᵥᵢᵥₒ*), où Test F et Test A*ᵥᵢᵥₒ* ne sont pas pratiqué sur le corps humain.

9. Méthode selon l'une quelconque des revendications précédentes, comprenant les étapes de :
a) engagement de composés candidats dans un Test B et/ou un Test C, puis pré-sélection des candidats répondant positivement audit Test B et/ou audit Test C ;
b) engagement des composés candidats issus de l'étape a) dans un Test A, avantageusement un test *in vitro* pour mesurer la cinétique de secrétion d'apoB (Test A*ᵥᵢₜᵣₒ*), et sélection des candidats répondant positivement audit Test A;
c) sélection complémentaire éventuelle des composés candidats sélectionnés de l'étape b), par analyse des métabolites desdits candidats au moyen du Test D et/ou du Test E ;
d) engagement des composés candidats sélectionnés au cours des étapes a), b) et c) dans au moins un test de confirmation *in vivo* F et/ou A*ᵥᵢᵥₒ*, puis sélection des candidats répondant positivement au Test F et/ou au Test A*ᵥᵢᵥₒ*; et
e) confirmation des candidats sélectionnés au cours de l'étape précédente, par sélection au moyen d'un test *in vivo* de contrôle de la diminution des triglycérides du sang (Test H), où Test H n'est pas pratiqué sur le corps humain.

10. Utilisation de la méthode de criblage selon l'une quelconque des revendications précédentes comprenant au moins un Test A*ᵥᵢₜᵣₒ*, Test A*ᵥᵢᵥₒ* ou une combinaison de ceux-ci, pour déterminer la cinétique d'un paramètre lié à l'inhibition de la MTP par un composé candidat.
